# EUROPEAN PATENT SPECIFICATION

(11) **EP 1 189 923 B1**
(45) Date of publication and mention of the grant of the patent: **29.12.2004**
(21) Application number: 00938427.2
(22) Date of filing: 20.06.2000
(51) Int. Cl.: C07J 43/00, A61K 31/58, A61P 3/06

(54) **AROMATIC AND HETEROCYCLIC DERIVATIVES OF PHYTOSTEROLS AND/OR PHYTOSTANOLS FOR USE IN TREATING OR PREVENTING CARDIOVASCULAR DISEASE**
AROMATISCHE UND HETEROZYKLISCHE DERIVATE VON PHYTOSTEROLEN UND/ODER PHYTOSTANOLEN ZUR BEHANDLUNG VON GEFÄSSKRANKHEITEN
DERIVES AROMATIQUES ET HETEROCYCLIQUES DE PHYTOSTEROLS ET/OU DE PHYTOSTANOLS ET LEUR UTILISATION DANS LE TRAITEMENT OU LA PREVENTION DE L'AFFECTION CARDIO-VASCULAIRE

(30) Priority: 21.06.1999 US 337810
(43) Date of publication of application: 27.03.2002
(73) Proprietor: Forbes Medi-Tech Inc., Vancouver, British Columbia V6C 2T8 (CA)
(72) Inventor: KUTNEY, James, P., Vancouver, British Columbia V6L 3C7 (CA); MILANOVA, Radka, K., Vancouver, British Columbia V6G 1E1 (CA); DING, Yangbing, Vancouver, British Columbia V6T 1R9 (CA); CHEN, Honming, Vancouver, British Columbia V6T 1R9 (CA)
(74) Representative: Jones, Helen Marjorie Meredith
(86) International application number: PCT/CA2000/000731
(87) International publication number: WO 2000/078789

(56) References cited:
- EP-A- 0 430 078
- US-A- 2 586 438
- MOTOC C ET AL: "MESOMORPHIC PROPERTIES OF SOME SITOSTERYL ESTERS" MOLECULAR CRYSTALS AND LIQUID CRYSTALS,GB,GORDON AND BREACH, LONDON, vol. 53, no. 1/02, 1979, pages 69-76, XP000677557 ISSN: 0026-8941
- CHEMICAL ABSTRACTS, vol. 61, no. 8, 12 October 1964 (1964-10-12) Columbus, Ohio, US; abstract no. 9556g, E. M. BALONOVA ET AL: "Esters of beta.-sitotosterol" XP002150764 & ZH. OBSHCH. KHIM., vol. 34, no. 7, 1964, pages 2157-2158,
- CHEMICAL ABSTRACTS, vol. 113, no. 17, 22 October 1990 (1990-10-22) Columbus, Ohio, US; abstract no. 152804, N. S. HABIB ET AL: "Antilipemic agents. III. Synthesis of some heterocyclic derivatives of beta.-sitosterol" page 818; column 1; XP002150765 & ARCH. PHARM., vol. 323, no. 7, 1990, pages 401-404, weinheim, de
- DATABASE BIOSIS [Online] BIOSCIENCES INFORMATION SERVICE, PHILADELPHIA, PA, US; 1992 STEINMETZ A ET AL: "DRUG THERAPY OF HYPERLIPOPROTEINEMIA" Database accession no. PREV199294065959 XP002150767 & INTERNIST, vol. 33, no. 1, 1992, pages 44-53, ISSN: 0020-9554
- DATABASE BIOSIS [Online] BIOSCIENCES INFORMATION SERVICE, PHILADELPHIA, PA, US; 1995 SCHWANDT P ET AL: "Drug treatment of hypercholesterolemia." Database accession no. PREV199698611586 XP002150768 & WIENER KLINISCHE WOCHENSCHRIFT, vol. 107, no. 18, 1995, pages 544-548, ISSN: 0043-5325

## Description

### FIELD OF THE INVENTION

This present invention relates to the field of phytosterols and phytostanols and their use in treating and preventing cardiovascular disease and other disorders.

### BACKGROUND OF THE INVENTION

While recent advances in science and technology are helping to improve quality and add years to human life, the prevention of atherosclerosis, the underlying cause of cardiovascular disease ("CVD") has not been sufficiently addressed. Atherosclerosis is a degenerative process resulting from an interplay of inherited (genetic) factors and environmental factors such as diet and lifestyle. Research to date suggest that cholesterol may play a role in atherosclerosis by forming atherosclerotic plaques in blood vessels, ultimately cutting off blood supply to the heart muscle or alternatively to the brain or limbs, depending on the location of the plaque in the arterial tree (1,2). Overviews have indicated that a 1% reduction in a person's total serum cholesterol yields a 2% reduction in risk of a coronary artery event (3). Statistically, a 10% decrease in average serum cholesterol (e.g. from 6.0 mmol/L to 5.3 mmol/L) may result in the prevention of 100,000 deaths in the United States annually (4).

Sterols are naturally occurring compounds that perform many critical cellular functions. Phytosterols such as campesterol, stigmasterol and beta-sitosterol in plants, ergosterol in fungi and cholesterol in animals are each primary components of cellular and sub-cellular membranes in their respective cell types. The dietary source of phytosterols in humans comes from plant materials i.e. vegetables and plant oils. The estimated daily phytosterol content in the conventional western-type diet is approximately 60-80 milligrams in contrast to a vegetarian diet which would provide about 500 milligrams per day.

Phytosterols have received a great deal of attention due to their ability to decrease serum cholesterol levels when fed to a number of mammalian species, including humans. While the precise mechanism of action remains largely unknown, the relationship between cholesterol and phytosterols is apparently due in part to the similarities between the respective chemical structures (the differences occurring in the side chains of the molecules). It is assumed that phytosterols displace cholesterol from the micellar phase and thereby reduce its absorption or possibly compete with receptor and/or carrier sites in the cholesterol absorption process.

Over forty years ago, Eli Lilly marketed a sterol preparation from tall oil and later from soybean oil called Cytellin™ which was found to lower serum cholesterol by about 9% according to one report (5). Various subsequent researchers have explored the effects of sitosterol preparations on plasma lipid and lipoprotein concentrations (6) and the effects of sitosterol and campesterol from soybean and tall oil sources on serum cholesterols (7). A composition of phytosterols which has been found to be highly effective in lowering serum cholesterol is disclosed in US Patent Serial No. 5,770,749 to Kutney et al. and comprises no more than 70% by weight beta-sitosterol, at least 10% by weight campesterol and stigmastanol (beta-sitostanol). It is noted in this patent that there is some form of synergy between the constituent phytosterols, affording even better cholesterol-lowering results than had been previously achieved.

Despite the obvious and now well recorded advantages of phytosterols, not only in the treatment of CVD and its underlying conditions such as hypercholesterolemia, hyperlipidemia, atherosclerosis, hypertension, thrombosis but in the treatment of other diseases such as Type II diabetes, dementia cancer and aging, the administration of phytosterols and the incorporation thereof into foods, pharmaceuticals and other delivery vehicles has been complicated by the fact that they are highly hydrophobic (i.e.they have poor water solubility). The provision of a water-soluble phytosterol derivative which could be administered orally and which could be incorporated without further modification into delivery vehicles would be highly desirable and has not heretofore been achieved.

It is an object of the present invention to obviate or mitigate the above disadvantages.

### SUMMARY OF THE INVENTION

The present invention provides novel phytosterol and/or phtostanol derivatives, including salts thereof, and represented by the general formulae of claim 13.

The present invention also provides the use of the novel derivatives and other derivatives as defined in claim 1 in the manufacture of a composition for treating or preventing CVD and its underlying conditions including atherosclerosis, hypercholesterolemia, hyperlipidemia, hypertension, thrombosis, and related diseases such as Type II diabetes, as well as other diseases that include oxidative damage as part of the underlying disease process such as dementia, aging, and cancer by administering to an animal a composition which comprises one or more derivatives of phytosterols and/or phytostanols having the above noted formula and a pharmaceutically acceptable carrier.

The present invention further provides food, beverages and nutraceuticals supplemented with one or more derivatives of phytosterols and/or phytostanols having one of the formulae in claim 1.

The phytosterol/phytostanol derivatives and salts thereof used in the present invention have numerous advantages over non-modified phytosterol/stanol compositions previously known and described in the art. In particular, it has been found that solubility in aqueous solutions such as water is improved thereby allowig oral administration per se without any further enhancements or modifications. Accordingly, the derivatives can be prepared and used as such or they can be easily incorporated into foods, beverages,
pharmaceuticals and nutraceuticals regardless of whether these "vehicles" are water-based.

A second advantage of the derivatives of the present invention is that, depending on the choice of R3 group, there can be additive or synergistic therapeutic effect between the phytosterol/stanol component and the R3 group. These effects and other significant advantages are described in more detail below.

### BRIEF DESCRIPTION OF THE DRAWINGS

Some aspects of the present invention are further described by way of the following non-limiting drawings in which:
Figure 1 is a graph showing the effects of one of the derivatives of the present invention on plasma cholesterol levels in apo E-KO mice;
Figure 2 is a graph showing the effects of one of the derivatives of the present invention on plasma triglyceride levels in apo E-KO mice;
Figure 3 is a graph showing the effects of one of the derivatives of the present invention on the body weight of apo E-KO mice during a treatment protocol;
Figure 4 is a graph showing plasma nicotinic acid concentrations in apo E-KO mice;
Figure 5 is a graph showing plasma nicotinamide concentrations in apo E-KO mice; and
Figure 6 is a schematic showing a process of preparing di(3-pyridinemethoxy)stanoxyphosphate, one of the derivatives of the present invention.

The derivatives used in the present invention are represented by the following formulae: 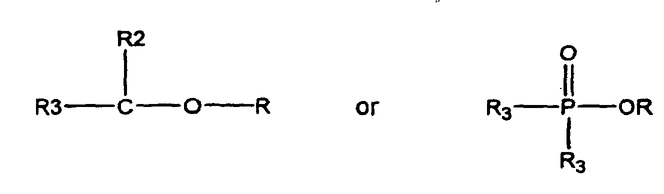 wherein R is a phytosterol or phytostanol moiety; R2 is oxygen or hydrogen (H2) and
R3 is an aromatic or heterocyclic unit. Each component of the derivative will be described below.

### Phytosterois/Phytostanois

As used herein, the term "phytosterol" includes all phytosterols without limitation, for example: sitosterol, campesterol, stigmasterol, brassicasterol, desmosterol, chalinosterol, poriferasterol, clionasterol and all natural or synthesized forms and derivatives thereof, including isomers. The term "phytostanol" includes all saturated or hydrogenated phytosterols and all natural or synthesized forms and derivatives thereof, including isomers. It is to be understood that modifications to the phytosterols and phytostanols i.e. to include side chains also falls within the purview of this invention. It is also to be understood that, when in doubt throughout the specification, the term "phytosterol" encompasses both phytosterol and phytostanol i.e. the terms may be used interchangeably unless otherwise specified.

The phytosterols and phytostanols for use in forming derivatives in accordance with this invention may be procured from a variety of natural sources. For example, they may be obtained from the processing of plant oils (including aquatic plants) such as com oil and other vegetable oils, wheat germ oil, soy extract, rice extract, rice bran, rapeseed oil, sesame oil and fish oils. Without limiting the generality of the foregoing, it is to be understood that there are other sources of phytosterols and phytostanols such as marine animals from which the composition of the present invention may be prepared. US Patent Serial No. 4,420,427 teaches the preparation of sterols from vegetable oil sludge using solvents such as methanol. Alternatively, phytosterols and phytostanols may be obtained from tall oil pitch or soap, by-products of forestry practises as described in US Patent Serial No.5,770,749, incorporated herein by reference.

In a most preferred form, the derivative of the present invention is formed with naturally-derived or synthesized sitostanol or with naturally derived or synthesized campestanol or mixtures thereof.

### R3―The Aromatic or Heterocyclic Unit

As used herein, R3 is defined as comprised of an aromatic unit or heterocyclic unit. In one embodiment of this invention, wherein R3 is aromatic, the unit is a benzene or substituted benzene ring (1) where the substituents R1-R5 are either hydrogen or alkyl. The alkyl groups are comprised of the family C-1 to C-6.

In another embodiment of the invention, wherein R3 is a heterocyclic unit, the heterocyclic ring may be a pyridine or substituted pyridine (2-4) where the substituents R1-R4 are either hydrogen or alkyl. The alkyl groups are comprised of the family C1-C6. As shown below, the pyridine unit can be attached at C3 (2), C2 (3) or at C4 (4). Most preferably, R3 is pyridine beta-carboxylic acid, commonly known as niacin or nicotinic acid, or derivative thereof.

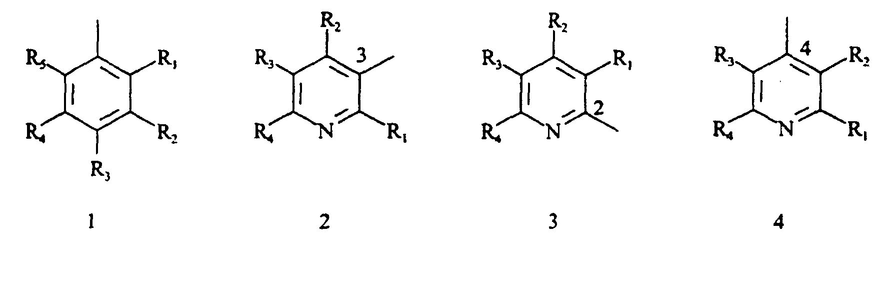

In another embodiment of the invention, wherein R3 is a heterocyclic unit, the heterocyclic ring may be a quinoline or substituted quinoline (5-7) where the substituents R1-R6 are either hydrogen or alkyl. The alkyl groups are comprised of the family C1-C6. As shown below, the quinoline unit can be attached at C2 (5), C3 (6) and C4 (7).

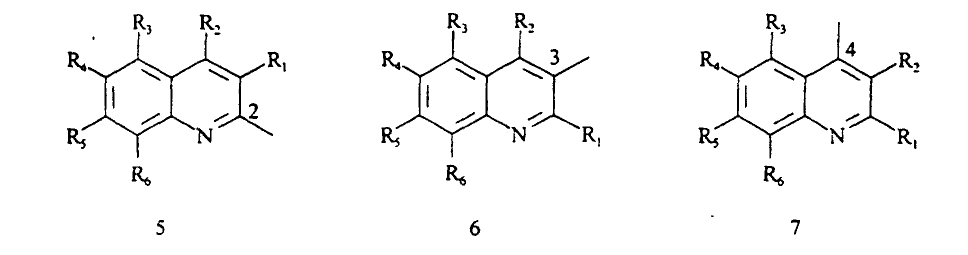

In another embodiment of the invention, wherein R3 is a heterocyclic unit, the heterocyclic ring may be an isoquinoline or substituted isoquinoline (8-10) where the substituents R1-R6 are either hydrogen or alkyl. The alkyl groups are comprised of the family C1-C6. As shown below, the isoquinoline unit can be attached at C3 (8), C1 (9) or C4 (10).

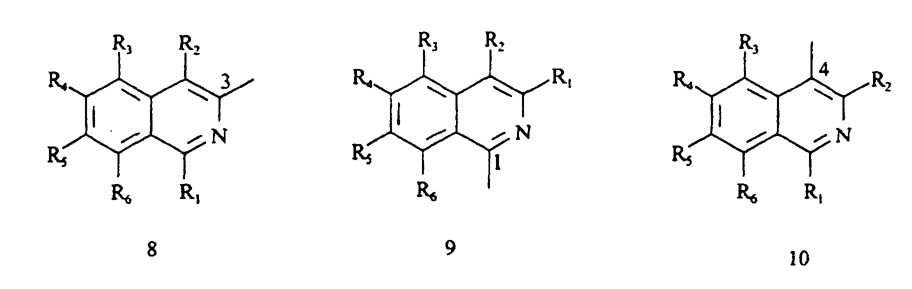

In another embodiment of the invention, wherein R3 is a heterocyclic unit, the heterocyclic ring may be an indole or substituted indole (11-12) where R1-R5 are either hydrogen of alkyl. The alkyl groups are comprised of the family C1-C6. As shown below, the indole unit can be atttached at C2 (11) or C3 (12).

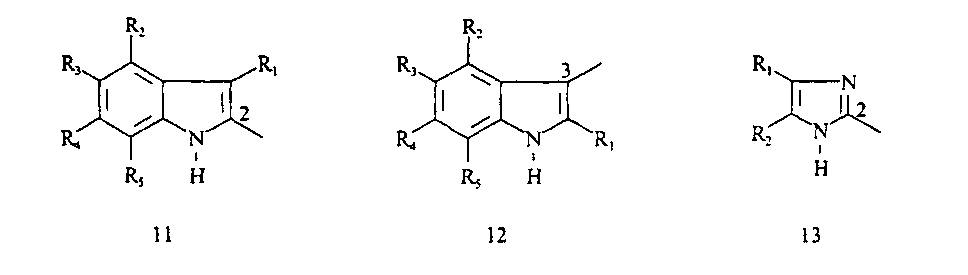

In another embodiment of the invention, wherein R3 is a heterocyclic unit, the heterocyclic ring may be an imidazole or a substituted imidazole (13-15) where R1-R2 are either hydrogen or alkyl. The alkyl groups are comprised of the family C1-C6. As shown below, the imidazole unit can be attached at C2 (13) or C4 (14) or C5 (15).

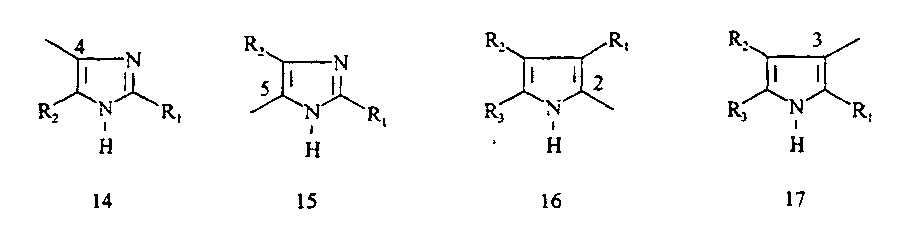

In another embodiment of the invention, wherein R3 is a heterocyclic unit, the heterocyclic ring may be a pyrrole ring or a substituted pyrrole (16-17) where R1-R3 are either hydrogen or alkyl. The alkyl groups are comprised of the family C1-C6. As shown, the pyrrole unit can be attached at C2 (16) or C3 (17).

In another embodiment of the invention, wherein R3 is a heterocyclic unit, the heterocyclic ring may be a furan or substitued furan (18-19) where R1-R3 are either hydrogen or alkyl. The alkyl groups are comprised of the family C1-C6. As shown belwo, the furan ring can be attached at C2 (18) or C3 (19).

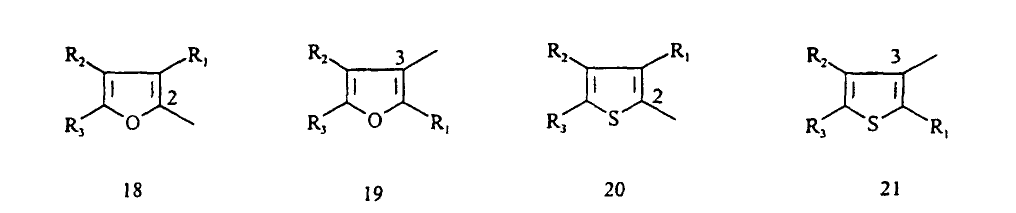

In another embodiment of the invention, wherein R3 is a heterocyclic unit, the heterocyclic ringmay be a thiophene or substituted thiophene (20-21) where R1-R3 are either hydrogen or alkyl. The alkyl groups are of the family C1-C6. As shown below, the thiophene ring can be attached at C2 (20) or C3 (21).

The novel phytosterol derivatives formed in accordance with the present invention may be either esters or ethers depending on the nature of R2. Simply put, when R2 is oxygen, the resultant derivative is an ester and when R2 is H2, the derivative is an ether.

### Derivative Formation

The general formula of the present invention noted above defines phytosterol/phytostanol-based esters or ethers.

### a) Ester Formation

To form the ester derivative comprising phytosterol and/or phytostanol, one or more aromatic or heterocyclic carboxylic acids (derived from the 21 sample formulae noted above) are condensed with the phytosterol and/or phytostanol under suitable reaction conditions. These conditions are the same as those used in other common esterification reactions such as the Fisher esterification process in which the carboxylic acid components and the alcohol component are allowed to react in the presence of a suitable acid catalyst such as mineral acid, sulfuric acid, phosphoric acid, p-toluenesulfonic acid. The organic solvents generally employed in such esterification reactions are ethers such as diethyl ether, tetrahydrofuran, or benzene, toluene or similar aromatic solvents and the temperatures can vary from room to elevated temperatures depending on the reactivity of the reactants undergoing the reaction.

In an alternative process to form the ester denvative comprising phytosterol and or phytostanol, the aromatic or heterocyclic carboxylic acid derivatives such as the corresponding acid halide (chloride, bromide or iodide) or the corresponding acid anhydride are condensed with the phytosterol/phytostanol under suitalbe reaction conditions. In general, such condensation reactions are conducted in an organic solvent such as diethyl ether, tetrahydrofuran, or benzene, toluene or similar aromatic solvents. Depending on the nature and reactivity of the reactants, the reaction temperatures may vary from low (-15 C) to elevated temperatures.

### b) Ether Formation

To form the ether derivative, the classsical method of ether formation is employed. This involves formation of the alkoxide form of the phytosterol/phytostanol by reaction of the latter with a strong base such a sodium hydride, sodium amide, sodium alkoxide, lithium diisopropyl amide, in an anhydrous inert solvent such as diethyl ether, tetrahydrofuran or benzene, toluene or similar aromatic solvent. The resulting anionic phytosterol/phytostanol derivative is then condensed with a suitable derivative of the parent aromatic or heterocyclic unit shown as R3 in the above-noted formula. In general, the latter derivative is formed by reduction of the carboxylic acid function, attached to the aromatic or heterocyclic ring, to the corresponding primary alcohol and the latter is transformed to the halide by reaction of the alcohol with thionyl chloride, phosphorus trihalide, phosphorus pentahalide or by treatment with mineral acid. Condensation of these two units in an inert anhydrous solvent such as diethyl ether, tetrahydrofuran or benzene, toluene or similar aromatic solvent generally at room or elevated temperature, results in the desired ether formation.

In an alternative process, the anionic phytosterol/phytostanol derivative formed as noted above, can be condensed with a suitable ester derivative of the alcohol obtained in the reduction of the carboxylic acid function in the aromatic or heterocyclic unit. Such a suitable ester can be obtained by reacting the latter alcohol with a benzoyl halide, 3,5-dinitrobenzoyl halide, p-toluensulfonyl halide or similar under classical esterification conditions noted above. Subsequent condensation of the anionic phytosterol.phytostanol derivative with the ester derivative of the aromatic or heterocyclic unit, in an inert anhydrous solvent such as diethyl ether, tetrahydrofuran or benzene, toluene or similar aromatic solvent, at room to elevated temperatures, affords the desired ether derivatives.

### c) Salt Formation

Since the majority of the ester and ether derivatives of the phytosterol/phytostanol system involve an attachment of a basic nitrogen heterocyclic component shown as R3 in the above-noted general formula (examples 2-10 and 13-15), it is possible to form salt derivatives of these compounds. These salts are expected to be more water soluble than the corresponding parent bases and therefore their efficacy and evaluation both *in vitro* and *in vivo* can be much improved.

Salt formation of these basic nitrogen derivatives can be readily performed by treatment of the parent base with a series of acids (for example, mineral acids, sulfuric acid, phosphoric acid, acetic and related carboxylic acids).

### d) Reduction by Catalytic (Hydrogenation) and Chemical Methods

Optionally, the phytosterol derivatives of the present invention or the constituent moieties thereof (either the phytosterol or the aromatic/heterocylic unit) prior to or after derivative formation may be hydrogenated or saturated. The hydrogenation of heterocyclic ring systems to the partially or fully reduced analogues is a well known process. For example, the catalytic and/or chemical reduction of pyridine rings to the corresponding dihydropyridine units (22) or the fully reduced piperidine units (23) is readily accomplished under an atmosphere of hydrogen and a metal catalyst such as platinum, palladium or Raney Nickel. In general, this reduction is performed in an organic solvent such as ethanol, ethyl acetate or similar media and either under atmospheric pressure or at a low pressure (3-5 psi) at room temperature or slightly elevated temperatures.

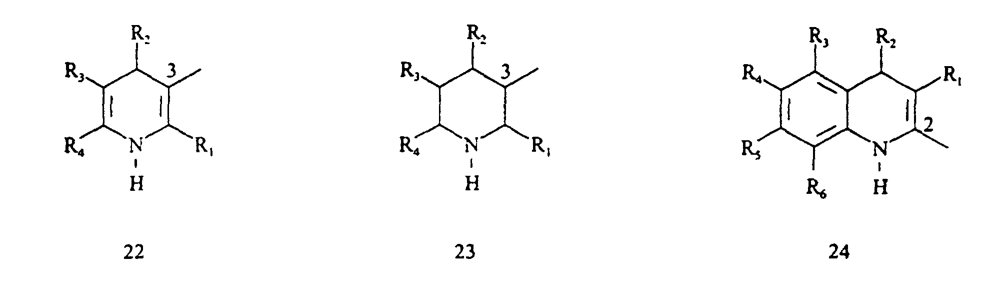

The chemical reductions of such systems involve reduction with a family of "hydride" reagents such as sodium borohydride, lithium aluminum hydride and their analogues. These reductions are generally performed in an anhydrous inert medium involving ethyl ether, tetrahydrofuran, dioxane, or benzene, toluene or similar aromatic solvents at room to reflux temperatures.

Similar reductions of the other heterocyclic systems such as quinoline, and isoquinoline to the corresponding dihydro- (24,25) and tetrahydro- (26,27) analogues can be achieved by the same catalytic and/or chemical processes.

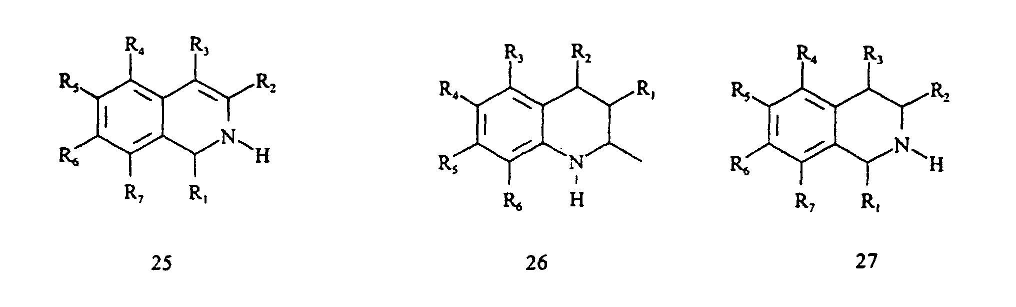

Similar catalytic or chemical processes can be applied to all of the phytosterol analogues of the present invention. Accordingly, the present invention includes within its scope all fully or partially reduced derivatives including the embodiment wherein R3 comprises a partially or fully reduced heterocyclic unit and/or wherein the phytosterol moiety is fully or partially hydrogenated.

### e) Potential Advantages of Novel Phytosterol Analogues as Cholesterol Lowering Agents

The novel derivatives of the present invention, wherein an aromatic and/or heterocyclic unit shown as R3 in the above-noted general formula is attached to the phytosterol moiety affords many dietary and therapeutic advantages when compared to the use of phytosterols/phytostanols without such attachment. First and foremost, efficacy of the derivative in lowering serum cholesterol, in addition to other therapeutic uses, is enhanced as compared to the administration of "unattached" phytosterols. It is very likely that there is even a synergistic or additive effect between the phytosterol moiety and the aromatic/heterocyclic unit. Secondly and equally importantly, the formation of these derivatives reduces or eliminates harmful side-effects of administering some aromatic/heterocylic units (for example, niacin) while preserving their therapeutic effects. Although the mechanism of action of these novel derivatives in lowering serum cholesterol is unclear, it is likely that both intestinal cholesterol absorption is blocked i.e. at the enterocyte by the "intact" derivative and in addition, that at least some of the moieties of the derivative are individually absorbed to lower cholesterol systemically. It is likely that the nature of the attachment of the heterocyclic unit to the phytosterol/phytostanol structure dictates the stability of the derivative in the intestine. Although we do not wish to be limited to any one proposed mechanism of action, it is believed that if the derivative is an ester (wherein R2 in the general formula is oxygen), then hyrolysis by esterase enzymes within the body or the acidic or the alkaline nature within the stomach and/or the intestinal tract can remove the R3 unit by hydrolysis thereby releasing the free moieties (phytosterol and niacin) to act independently. These moieties would then be absorbed and systemically lower cholesterol. On the other hand, it is believed that if the derivative is an ether (wherein R2 in the general formula is hydrogen), hydrolysis is unlikely since hydrolysis of the ether linkage generally requires acidic catalysts at elevated temperatures. It is possible that an optimal composition for lowering serum cholesterol may comprise both esters and ethers of the derivatives of the present invention.

A third advantage of the novel derivatives of the present invention is enhanced solubility. This is particularly important in the manufacture of "delivery" vehicles as described further below.

In a preferred form, R3 is a pyridine/dihydropyridine unit. Although other R3 units are effective in forming the derivatives of the present invention, pyridine/dihydropyridine units have been found to form phytosterol derivatives which are surprisingly effective in lowering serum cholesterol. One of the most preferred pyridine units, nicotinic acid (niacin) has been used for many years as a cholesterol lowering agent which exhibits potent (20-30%) elevation of the "good" HDL cholesterol. Unfortunately, its use is limited by undesirable side effects (J.D. Alderman, *Am. J. Cardiol*. 64, 725 (1987); H. van den Berg, *Eur*.*J*. *Clin. Nutr.* 51, Suppl.1, S64 (1997). These side effects include severe liver damage and skin disorders. What has been found within the scope of the present invention, is that the formation of a derivative as defined herein, and wherein R3 is niacin, results in a compound having at least as good or better therapeutic effects than each of niacin and the phytosterol moiety alone coupled with a reduction in harmful side effects normally attendant with the use of niacin alone.

### Delivery Systems

Although it is fully contemplated within the scope of the present invention that the derivatives may be administered to animals, particularly humans, directly and without any further modification, it is possible to take further steps to enhance delivery and ensure even distribution throughout the food, beverage, pharmaceutical, nutraceutical and the like to which they are added. Such enhancement may be achieved by a number of suitable means such as, for example, solubilizing or dispersing the derivatives to form emulsions, solutions and dispersions or self-emulsifying systems; lyophilizing, spray drying, controlled precipitating, or a combination thereof; forming solid dispersions, suspensions, hydrated lipid systems; forming inclusion complexations with cyclodextrins; and using hydrotopes and formulations with bile acids and their derivatives.

Each of the techniques which may be used in accordance with the present invention are described below.

### Emulsions

Emulsions are finely divided or colloidal dispersions comprising two immiscible phases, e.g. oil and water, one of which (the internal or discontinuous phase) is dispersed as droplets within the other (external or discontinuous phase). Thus an oil-in-water emulsion consists of oil as the internal phase, and water as the discontinuous or external phase, the water-in-oil emulsion being the opposite. A wide variety of emulsified systems may be formed which comprise phytosterols or phytostanols derivatives including standard emulsions, microemulsions and those systems which are self-emulsifying (emulsify on exposure to agitated aqueous fluids such as gastric or intestinal fluids).

Generally, emulsions may include oil and water phases, emulsifiers, emulsion stabilizers and optionally preservatives, flavouring agents, pH adjusters and buffers, chelating agents, antifoam agents, tonicity adjusters and anti-oxidants. Suitable emulsifiers
(wherein bracketed numerals refer to the preferred HLB values) include: anionic surfactants such as alcohol ether sulfates, alkyl sulfates (30-40), soaps (12-20) and sulfosuccinates; cationic surfactants such as quaternary ammonium compounds; zwitterionic surfactants such as alkyl betaine derivatives; amphoteric surfactants such as fatty amine sulfates, difatty alkyl triethanolamine derivatives (16-17); and nonionic surfactants such as the polyglycol ether derivatives of aliphatic or cycloaliphatic alcohols, saturated fatty acids and alkyphenols, water-soluble polyethyleneoxy adducts onto polypropylene glycol and alkyl polypropylene glycol, nonylphenol polyethoxyethanols, castor oil polyglycol ethers, polypropylene/polyethylene oxide adducts, tributylphenoxy-polyethoxyethanol, polyethylene glycol, octylphenoxy-polyethoxyethanol, lanolin alcohols, polyoxyethylated (POE) alkyl phenols(12-13), POE fatty amides, POE fatty alcohol ethers, POE fatty amines, POE fatty esters, poloxamers (7-19), POE glycol monoethers (13-16), polysorbates (17-19) and sorbitan esters (2-9). This list is not intended to be exhaustive as other emulsifiers are equally suitable.

Appropriate emulsion stabilizers include, but are not limited to, lyophilic colloids such as polysaccharides, acacia, agar, alginic acid. carrageenan, guar gum, karaya gum, tragacanth, xanthan gum; amphoterics (e.g. gelatin) and synthetic or semi-synthetic polymers (e.g. carbomer resins, cellulose ethers and esters, carboxymethyl chitin, polyethylene glycol-n (ethylene oxide polymer H(OCH2CH2)nOH); finely divided solids including clays (e.g. attapulgite, bentonite, hectorite, kaolin, magnesium aluminum silicate and montmorillonite), microcrystalline cellulose oxides and hydroxides (e.g. aluminum hydroxide. magnesium hydroxide and silica); and cybotactic promoters/gellants (including amino acids, peptides, proteins lecithin and other phospholipids and poloxamers).

Suitable anti-oxidants for use in the formation of emulsions include: chelating agents such as citric acid, EDTA, phenylalanine, phosphoric acid, tartaric acid and tryptophane; preferentially oxidized compounds such as ascorbic acid, sodium bisulfite and sodium sulfite; water soluble chain terminators such as thiols and lipid soluble chain terminators such as alkyl gallates, ascorbyl palmitate, t-butyl hydroquinone, butylated hydroxyanisole, butylated hydroxytoluene, hydroquinone, nordihydroguaiaretic acid and alpha-tocopherol. Suitable preservatives, pH adjustment agents, and buffers, chelating agents, osmotic agents, colours and flavouring agents are discussed hereinbelow under "Supensions", but are equally applicable with respect to the formation of emulsions.

The general preparation of emulsions is as follows: the two phases (oil and water) are separately heated to an appropriate temperature, the same in both cases, generally 5-10°C above the melting point of the highest melting ingredients in the case of a solid or semi-solid oil, or where the oil phase is liquid, a suitable temperature as determined by routine experimentation). Water-soluble components are dissolved in the aqueous (water) phase and oil-soluble components, are dissolved in the oil phase. To create an oil-in water emulsion, the oil phase is vigorously mixed into the aqueous phase to create a suitable dispersion and the product is allowed to cool at a controlled rate with stirring. A water-in-oil emulsion is formed in the opposite fashion i.e. the water phase is added to the oil phase. When hydrophilic colloids are a part of the system as emulsion stabilizers, a phase inversion technique may be employed whereby the colloid is mixed into the oil phase rather than the aqueous phase, prior to addition to the aqueous phase. In using any phytosterol or phytostanol derivatives, it is preferred to add these to the oil phase prior to heating.

Microemulsions, characterized by a particle size at least an order of magnitude smaller (10-100 nm) than standard emulsions and defined as "a system of water, oil and amphiphile which is a single optically isotropic and thermodynamically stable liquid" (8), may also be formed comprising phytosterol or phytostanol derivatives. In a preferred form, the microemulsion comprises a surfactant or surfactant mixture, a co-surfactant (usually a short chain alcohol) the chosen phytosterol or phytostanol derivatives, water and optionally other additives.

This system has several advantages as a delivery system for the derivatives of the present invention. Firstly, microemulsions tend to be created spontaneously, that is, without the degree of vigorous mixing required to form standard emulsions. From a commercial perspective, this simplifies the manufacturing process. Secondly, microemulsions may be sterilized using microfiltration techniques without breaking the microstructure due to the small diameter of the microdroplets. Thirdly, microemulsions are highly thermodynamically stable. Fourthly, microemulsions possess high solubilizing power which is particularly important as they can further enhance the solubilization of the derivatives.

Surfactant or surfactant mixtures which are suitable for use in the formation of microemulsions can be anionic, cationic, amphoteric or non-ionic and possess HLB (hydrophile-lipophile balance) values within the range of 1-20, more preferably in the ranges 2-6 and 8-17. Especially preferred agents are non-ionic surfactants, selected from the group consisting of polyglycol ether derivatives of aliphatic or cycloaliphatic alcohols, saturated fatty acids and alkyphenols, water-soluble polyethyleneoxy adducts onto polypropylene glycol and alkyl polypropylene glycol, nonylphenol polyethoxyethanols, castor oil polyglycol ethers, polypropylene/polyethylene oxide adducts, tributylphenoxy-polyethoxyethanol, polyethylene glycol, octylphenoxy-polyethoxyethanol, lanolin alcohols, polyoxyethylated (POE) alkyl phenols (12-13), POE fatty amides, POE fatty alcohol ethers, POE fatty amines, POE fatty esters, poloxamers (7-19), POE glycol monoethers (13-16), polysorbates (10-17) and sorbitan esters (2-9).

There are a number of methods known and used by those skilled in the art for making microemulsions. In a preferred method of forming microemulsions of the present invention, a surfactant, a co-surfactant, and phytosterol or phytostanol derivative (predissolved in a suitable proportion of an appropriate oil) are mixed and then titrated with water until a system of desired transparency is obtained.

In a further preferred embodiment, the formation of microemulsions may be achieved by mixing the phytosterol or phytostanol derivatives with hydrotropic agents and food-grade surfactants (refer to 9).

### Solutions and Dispersions

Phytosterol or phytostanol derivatives may be dissolved or dispersed in a suitable oil vehicle, with or without additional excipients, and used in this form, for example, in general food usage, in basting meats and fish, and for incorporation into animal feeds.

Suitable solubilizing agents include all food grade oils such as plant oils, marine oils (such as fish oil) and vegetable oils, monoglycerides, diglycerides, triglycerides, tocopherols and the like and mixtures thereof.

### Self-Emulsifying Systems

Phytosterol or phytostanol derivatives may be mixed with appropriate excipients, for example, surfactants, emulsion stabilizers (described above) and the like, heated (if necessary) and cooled to form a semi-solid product capable of forming a spontaneous emulsion on contact with aqueous media. This semi-solid product may be used in numerous other forms such as filler material in two-piece hard or soft gelatin capsules, or may be adapted for use in other delivery systems.

### Solid Dispersions

An alternative means of further increasing the solubility/dispersability of the derivatives in accordance with the present invention involves the use of solid dispersion systems. These dispersions may include molecular solutions (eutectics), physical dispersions or a combination of both.

For example, solid dispersions may typically be prepared by utilizing water-soluble polymers as carriers. Without limitation, these carriers may include, either alone or in combination: solid grade polyethylene glycols (PEG's), with or without the addition of liquid grade PEG's; polyvinylpyrrolidones or their co-polymers with vinyl acetate and cellulose ethers and esters. Other excipients, such as additional members of the glycol family e.g. propylene glycol, polyols, e.g. glycerol etc.. may also be included in the dispersions.

Solid dispersions may be prepared by a number of ways which are familiar to those in the art. These include, without limitation, the following methods:
(a) fusing the ingredients, followed by controlled cooling to allow solidification and subsequent mechanical grinding to produce a suitable powder. Alternatively, the molten (fused) dispersion may be sprayed into a stream of cooled air in a spray drier to form solid particles (prilling) or passed through an extruder and spheroniser to form solid masses of a controlled particle size. In a further alternative, the molten dispersion is filled directly into two-piece hard gelatin capsules;
(b) dissolving the ingredients in a suitable solvent system (organic, mixed organic, organic-aqueous) and then removing the solvents e.g. by evaporating at atmospheric pressure or in vacuo, spray drying, lyophilizing and the like; or, in a variation of the foregoing, and
(c) dissolving the ingredients in a suitable solvent system, subsequently precipitating them from solution by the use of an immiscible solvent in which the ingredients have little or no solubility, filtration, removing the solvent, drying and optionally grinding to provide a suitable powder form.

### Suspensions

Suspensions, which may be used to enhance further the solubility and/or dispersability of the derivatives , comprise a solid, perhaps finely divided, internal phase dispersed in an oily or aqueous external phase (the vehicle). In addition, the solid internal phase may be added to an emulsion as described above during its' formation to produce a delivery system having properties common to both suspensions and emulsions.

Numerous excipients, which are commonly used in the art, may be suitable for producing a suspension within the scope of the present invention. Typically, a suspension comprises an oily or aqueous vehicle, the dispersed (suspended) internal phase, dispersing and/or wetting agents (surfactants), pH adjustment agents/buffers, chelating agents, antioxidants, agents to adjust ionic strength (osmotic agents) colours, flavours, substances to stabilize the suspension and increase viscosity (suspending agents) and preservatives.

Appropriate vehicles include, but are not limited to: water, oils, alcohols, polyols, other edible or food grade compounds in which the phytosterol composition is partially or not soluble and mixtures thereof. Appropriate dispersing agents include, but are not limited to: lecithin; phospholipids; nonionic surfactants such as polysorbate 65, octoxynol-9, nonoxynol-10, polysorbate 60, polysorbate 80, polysorbate 40, poloxamer 235, polysorbate 20 and poloxamer 188; anionic surfactants such as sodium lauryl sulfate and docusate sodium; fatty acids, salts of fatty acids, other fatty acid esters, and mixtures thereof.

Agents/buffers for pH adjustment include citric acid and its salts, tartaric acid and its salts, phosphoric acid and its salts, acetic acid and its salts, hydrochloric acid, sodium hydroxide and sodium bicarbonate. Suitable chelating agents include edetates (disodium, calcium disodium and the like), citric acid and tartaric acid. Suitable antioxidants include ascorbic acid and its salts, ascorbyl palmitate, tocopherols (especially alpha-tocopherol), butylated hydroxytoluene, butylated hydroxyanisole, sodium bisulfite and metabisulfite. Suitable osmotic agents include monovalent, divalent and trivalent electrolytes, monosaccharides and disaccharides. Suitable preservatives include parabens (Me, Et, Pr, Bu and mixtures thereof), sorbic acid, thimerosal, quaternary ammonium salts, benzyl alcohol, benzoic acid, chlorhexidine gluconate and phenylethanol. Colours and flavours may be added as desired and may be selected from all natural, nature-identical and synthetic varieties.

### Hydrated Lipid Systems

In a further embodiment of the present invention, the solubility/dispersability of the derivtaives of the present invention may be further enhanced by the formation of phospholipid systems such as liposomes and other hydrated lipid phases, by physical inclusion. This inclusion refers to the entrapment of molecules without forming a covalent bond and is widely used to improve the solubility and subsequent dissolution of active ingredients.

Hydrated lipid systems, including liposomes, can be prepared using a variety of lipid and lipid mixtures, including phospholipids such as phosphatidylcholine (lecithin), phosphodiglyceride and sphingolipids, glycolipids, and the like. The lipids may preferably be used in combination with a charge bearing substances such as charge-bearing phospholipids, fatty acids, and potassium and sodium salts thereof in order to stabilize the resultant lipid systems. A typical process of forming liposomes is as follows:
1) dispersion of lipid or lipids and the phytosterols or phytostanols or mixtures thereof and the tocotrienol component in an organic solvent (such as chloroform, dichloromethane, ether, ethanol or other alcohol, or a combination thereof). A charged species may be added to reduce subsequent aggregation during liposome formation.
   Antioxidants (such as ascorbyl palmitate, alpha-tocopherol, butylated hydroxytoluene and butylated hydroxyanisole) may also be added to protect any unsaturated lipids, if present;
2) filtration of the mixture to remove minor insoluble components;
3) removal of solvents under conditions (pressure, temperature) to ensure no phase separation of the components occur;
4) hydration of the "dry" lipid mixture by exposure to an aqueous medium containing dissolved solutes, including buffer salts, chelating agents, cryoprotectorants and the like; and
5) reduction of liposome particle size and modification of the state of lamellarity by means of suitable techniques such as homogenization, extrusion etc..

Any procedure for generating and loading hydrated lipid with active ingredients, known to those skilled in the art, may be employed within the scope of this invention. For example, suitable processes for the preparation of liposomes are described in references 10 and 11, both of which are incorporated herein by reference. Variations on these processes are described in US Patent Serial No. 5,096,629 which is also incorporated herein by reference.

US Patent Serial No. 4,508,703 (also incorporated herein by reference) describes a method of preparing liposomes by dissolving the amphiphillic lipidic constituent and the hydrophobic constituent to form a solution and thereafter atomizing the solution in a flow of gas to produce a pulverent mixture.

### Cyclodextrin Complexes

Cyclodextrins are a class of cyclic oligosaccharide molecules comprising glucopyranose sub-units and having a toroidal cylindrical spatial configuration. Commonly available members of this group comprise molecules containing six (alpha-cyclodextrin), seven (beta-cyclodextrin) and eight (gamma-cylcodextrin) glucopyranose molcules, with the polar (hydrophilic) hydroxyl groups oriented to the outside of the structure and the apolar (lipophilic) skeletal carbons and ethereal oxygens lining the interior cavity of the toroid. This cavity is capable of accomodating (hosting) the lipophilic moiety of an active ingredient (the guest molecule, here the derivative of the present invention) by bonding in a non-covalent manner to form an inclusion complex.

The external hydroxyl substituents of the cyclodextrin molecule may be modified to form derivatives having improved solubility in aqueous media along with other desired enhancements, such as lowered toxicity, etc.. Examples of such derivatives are: alkylated derivatives such as 2,6-dimethyl-beta-cyclodextrin; hydroxyalkylated derivatives such as hydroxypropyl-beta-cyclodextrin; branched derivatives such as digiucosly-beta-cyclodextrin; sulfoalkyl derivatives such as sulfobutylether-beta-cyclodextrin; and carboxymethylated derivatives such as carboxymethyl-beta-cylcodextrin. Other types of chemical modifications, known to those in the art, are also included within the scope of this invention.

The cyclodextrin complex often confers properties of improved solubility, dispersability, stability (chemical, physical and microbiological), bioavailability and decreased toxicity on the guest molecule (here, the derivative of the present invention).

There are a number of ways known in the art to produce a cyclodextrin complex. Complexes may be produced, for example, by using the following basic methods: stirring the one or more derivatves into an aqueous or mixed aqueous-organic solution of the cyclodextrin, with or without heating: kneading, slurrying or mixing the cyclodextrin and the present composition in a suitable device with the addition of an appropriate quantity of aqueous, organic or mixed aqueous-organic liquid, with or without heating; or by physical admixture the cylcodextrin and the composition of the present invention using a suitable mixing device. Isolation of the inclusion complex so formed may be achieved by co-precipitation, filtration and drying; extrusion/spheronisation and drying; subdivision of the moist mass and drying; spray drying; lyophilization or by other suitable techniques depending on the process used to form the cyclodextrin complex. A further optional step of mechanically grinding the isolated solid complex may be employed.

These cyclodextrin complexes further enhance the solubility and dissolution rate and increase the stability of the derivatives. For a review of cyclodextrin complexation, please refer to 12.

### Complexation with Bile Salts

Bile acids, their salts and conjugated derivatives, suitably formulated, may be used to solubilize the derivatives of the present invention, thereby improving the solubility and dispersion characteristics of these compositions. Examples of suitable bile acids include: cholic acid, chenodeoxycholic acid, deoxycholic acid, dehydrocholic acid, and lithocholic acid. Examples of suitable bile salts include: sodium cholate, sodium deoxycholate and their other salt forms. Examples of suitable conjugated bile acids include: glycochenodeoxycholic acid, glycholic acid, taurochenodeoxycholic acid, taurocholic acid, taurodeoxycholic acid and their salts.

A suitable system for further enhancing the solubility of the derivative of the present invention consists of one or more derivatives plus one or more bile acids, salts or conjugated bile acids. Further materials may be added to produce formulations having additional solubilization capacity. These materials include, but are not limited to: phospholipids, glycolipids and monoglycerides. These ingredients may be formulated either in the solid phase or by the use of suitable solvents or carrier vehicles, with appropriate isolation and, optionally, particle size reduction using techniques described hereinabove.

Since bile acids and their derivatives have an unpleasant taste and may be irritating to the mucous membranes of the stomach and upper regions of the gastro-intestinal tract, a suitable enteric coating may be applied to the solid formulation particulates, using techniques known to those skilled in the art. Typical enteric coatings include, inter alia: cellulose acetate phthalate, cellulose acetate trimellitate, hydroxypropylmethylcellulose phthalate, hydroxypropylmethylcellulose acetate succinate, polyvinylacetate phthalate, acrylate polymers and their derivatives (e.g. appropriate members of the Eudragit™ series), ethylcellulose or combinations thereof.

Additional excipients may be added to the coating formulation to modify membrane functionality or to aid in the coating process (e.g. surfactants, plasticisers, channeling agents, permeability modifiers and the like). Coating formulation vehicles may comprise aqueous or organic systems, or mixtures of both.

### Hydrotropic Complexation

Compounds which are capable of opening up the water structure associated with hydrophobic (lipophilic) and other molecules are referred to as hydrotropes. These compounds may be used to enhance further the aqueous solubility of the derivatives. Examples of hydrotopes include, inter alia, sodium benzoate, sodium hydroxybenzoates, sodium salicylate, nicotinamide, sodium nicotinate, sodium gentisate, gentisic acid ethanolamide, sodium toluates, sodium aminobenzoates, sodium anthranilate, sodium butylmonoglycolsulfate, resorcinol and the like.

Complex formation, which is non-covalent in nature, may be achieved by mixing one or more derivative and the hydrotope or mixtures thereof in a suitable liquid vehicle, which may be aqueous, organic or a combination of both. Additional excipients such as surfactants, polyols, disaccharides etc.. may be added to facilitate complexation or to aid in dispersability. The resultant complex is isolated as a dry powder by any process known in the art (co-precipitation and drying, evaporation of the liquid vehicle, spray drying, lyophilization etc..). Particle size may be reduced by any standard technique such as those described previously herein, if desired. The resultant hydrotope complex may be used without further modification or may be compounded into a variety of other formulations or vehicles as required.

### Methods of Use

The derivatives of the present invention may be administered to animals, in particular humans, directly and without further modification or may be treated to enhance further the solubility and/or dispersability of the composition as described in detail above. Alternatively, and optionally in conjunction with any one of these solubility and/or dispersability enhancement methods, the derivatives may be incorporated into various vehicles as described further below in order to treat and/or prevent CVD, its underlying conditions such as hypercholesterolemia, hyperlipidemia, arteriosclerosis, hypertension, thrombosis, related diseases such as Type II diabetes, as well as other diseases that include oxidative damage as part of the underlying disease process such as dementia, aging, and cancer. In populations, which are considered "high-risk" for CVD or any of the oxidation related disorders, it is contemplated that the derivatives of the present invention be used in primary, secondary and tertiary treatment programs.

Without limiting the generality of the foregoing, the derivatives of the present invention may be admixed with various carriers or adjuvants to assist in direct administration or to assist in the incorporation of the composition into foods, beverages, nutraceuticals or pharmaceuticals. In order to appreciate the various possible vehicles of the delivery of the derivatives, the list below is provided. The doses of the derivatives will vary depending upon, among other factors, the mode of delivery, the patient size and condition, the result to be achieved, as well as other factors known to those skilled in the art of food additives and medicinal agents. Generally, however, it is preferred that the derivatives of the present invention be administered to humans in a form comprising up to 6 grams of phytosterols and/or phytostanols per day. It will also be recognized that the provision of much larger daily doses of the derivatives are not harmful to the animal host, as excess will simply pass through normal excretory channels.

### 1) Pharmaceutical Dosage Forms:

It is contemplated within the scope of the present invention that the derivatives of the present invention may be incorporated into various conventional pharmaceutical preparations and dosage forms such as tablets (plain and coated) for use orally, bucally or lingually, capsules (hard and soft, gelatin, with or without additional coatings) powders, granules (including effervescent granules), pellets, microparticulates, solutions (such as micellar, syrups, elixirs and drops), lozenges, pastilles, ampoules, emulsions, microemulsions, ointments, creams, suppositories, gels, transdermal patches and modified release dosage forms together with customary excipients and/or diluents and stabilizers.

The derivatives of the present invention, adapted into the appropriate dosage form as described above may be administered to animals, including humans, orally, by injection (intravenously, subcutaneously, intra-peritoneally, intra-dermally or intramuscularly), topically or in other ways. Although the precise mechanism of action is unclear, the derivatives of the present invention, administered intra-venously, lower serum cholesterol. It is believed that certain phytosterol-based products may have, in addition to the role as an inhibitors of cholesterol absorption in the intestine, a systemic effect on cholesterol homeostasis through bile acid synthesis, enterocycte and biliary cholesterol excretion, bile acid excretion and changes in enzyme kinetics and cholesterol transport between various compartments within the body (PCT/CA97/00474 which was published on January 15, 1998).

### 2) Foods/Beverages/Nutraceuticals:

In another form of the present invention, the derivatives of the present invention may be incorporated into foods, beverages and nutraceuticals, including, without limitation, the following:
1) Dairy Products --such as cheeses, butter, milk and other dairy beverages, spreads and dairy mixes, ice cream and yoghurt;
2) Fat-Based Products--such as margarines, spreads, mayonnaise, shortenings, cooking and frying oils and dressings;
3) Cereal-Based Products-comprising grains (for example, bread and pastas) whether these goods are cooked, baked or otherwise processed;
4) Confectioneries--such as chocolate, candies, chewing gum, desserts, non-dairy toppings (for example Cool Whip™), sorbets, icings and other fillings;
5) Beverages-- whether alcoholic or non-alcoholic and including colas and other soft drinks, juice drinks, dietary supplement and meal replacement drinks such as those sold under the trade-marks Boost™ and Ensure™; and
6) Miscellaneous Products--including eggs and egg products, processed foods such as soups, pre-prepared pasta sauces, pre-formed meals and the like.

The derivatives of the present invention may be incorporated directly and without further modification into the food, nutraceutical or beverage by techniques such as mixing, infusion, injection, blending, dispersing, emulsifying, immersion, spraying and kneading. Alternatively, the derivatives may be applied directly onto a food or into a beverage by the consumer prior to ingestion. These are simple and economical modes of delivery.

### EXAMPLES

### Example 1. Preparation of stanol nicotinic acid ester.

Nicotinoyl chloride (6g) was suspended in chloroform (150ml) with a stanol mixture (11g) (campestanol: 36.4%; sitostanol: 62.3%). The mixture was stirred at room temperature, anhydrous pyridine (6ml) was added dropwise to the above prepared mixture. The stirring was continued for 3 hours. After the addition the mixture was concentrated to remove the solvent, and then to the remaining residue was added acetone (100ml). The resultant white precipitate was collected and recrystallized in an acetone:chloroform (2:1); 10g of white crystals were obtained.

### Example 2. Preparation of stanol nicotinate hydrochloride.

The stanol nicotinate prepared above (2g) was dissolved in dry ether (20ml), to which 36% HCl (1ml) was added under stirring at room temperature. A white precipitate was filtered off and washed with ether (10ml), dried, and then a white powder of stanol nicotinate hydrochloride (2g) was obtained.

### Example 3. Preparation of stanol nicotinoxyacetate

### Step a. Synthesis of stanol monochloroacetic ester.

Stanol mixture (4g) (campestanol (36.4%) and sitostanol (62.3%) in monochloroacetic acid (10ml) was heated to 120°C under stirring for 3 hours. After cooling down, water (50ml) was added to the reaction mixture, the precipitate was collected and washed with water (10ml), dried, yielded a white solid (4.5g).

### Step b. Synthesis of stanol nicotinoxyacetate.

Sodium nicotinate (0.5g) in dry DMF (10ml) was added to the above prepared stanol chloroacetate (1.5g), the mixture was heated to 140°C with stirring for 1 hour. After cooling down to room temperature, the mixture was poured into water (100ml), the off-white solid was collected, dried and weighed (1.8g).

### Example 4―Preparation of di(3-pyridinemethoxy)stanoxyphosphate

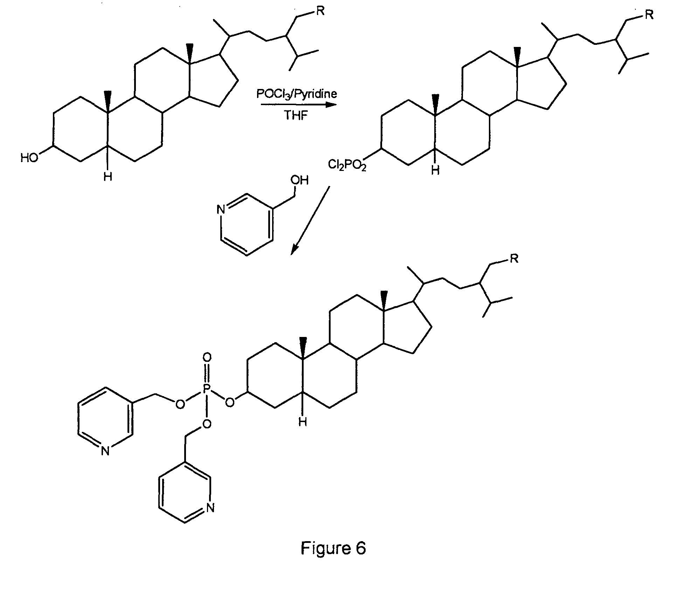

### Synthesis of di(3-pyridinemethoxy)stanoxyphosphate with reference to the Figure 6

### Step a. Synthesis of stanoxyphosphoric chloride.

Stanol mixture (4.0g) (campestanol: 36.4w/w%; sitostanol: 62.3w/w%) in THF (30ml) and pyridine (4ml) was added dropwise to POCl₃ (1.2g) in THF (10ml) under stirring at 0°C. After the addition, the mixture was stirred for 1 hour at room temperature. The resulted pyridine hydrochloride was filtered off, the filtrate was concentrated to dryness, and an oily mass (6.5g) was obtained.
¹HNMR (CDCl₃): 0.7 (3H, s), 4.7 (1H, m).
³¹PNMR (CDCl₃): ∼7ppm (s).

### Step b. Synthesis of di(3-pyridinemethoxy)stanoxyphosphate

Pyridine (5ml) was added to the above prepared oily mass, the mixture was stirred at 0°C and to which a solution of 3-pyridinemethanol (3.5ml) in pyridine (10ml) was introduced dropwise for 0.5hr. After the addition, the mixture was kept stirring for 1 hour, then poured into water (100ml), extracted with diethyl ether (50mlx2), dried over sodium sulfate (8g), concentrated and chromatographed on a silica gel column (50ml) with
eluting solvent (EtOAc:Hexanes=1:1). A white wax (3.7g) was finally obtained. Yield (start from Stanol mixture): 56.4%.
¹HNMR (CDCl₃): 8.60 (s, 4H), 7.70 (s, 2H), 7.30 (s, 2H), 5.0 (d, 4H), 4.30 (1H).

### Example 5―Cholesterol lowering effects of Derivatives in Mouse Model

### Materials and Methods

Forty 4-week male apolipoprotein E knockout (apo E-KO) mice were purchased from the Jackson Laboratory and housed in the animal facility at Research Center, BC Children's Hospital. PhytoNic (one of the derivatives of the present invention) was prepared by Forbes Med-Tech Inc. ("FMI") The parent material of PhyoNic, phyosterols and nicotinic acid were also provided by FMI. Cholesterol was purchased from Sigma and 9% (w/w) PicoLab mouse chow was purchased from Jameison's Pet Food distributor, Delta, BC Laboratory chemicals and reagents were prepared and used as previously described (13-16).

### Experimental design:

**Animals and diets:** Based on our own previous experiments, 8 animals in each group are sufficient to detect statistically significant treatment-related effects on plasma cholesterol levels. Thus, as outlined below, 40 male apo E-KO mice were divided into 5 groups of 8 with similar mean plasma cholesterol level and body weight. The animals were fed with the experimental diets for 14 weeks. Diet preparation was carried out based on previously published methods (13-16). The outline of experiemtal groups of mice and diets is presented in Table 1.

The animals were bled from the tail at base line and during the experiment for measurement of plasma lipid levels. At the end of the study, the mice were sacrificed using CO₂ gas and taking blood from the heart. The hearts and aorta were removed and fixed in formalin for the assessment of atherosclerotic lesions. Aliquots of plasma were sent to BRI laboratory, Burnaby, BC, for measurement of nicotinic acid and plant sterol concentrations.

**Table 1:**

| Experimental groups of mice and diets | |
|---|---|
| **Groups (n)** | **Treatment (types of diets)** |
| 1 (8) | Control mouse chow with 9% fat & 0.15% (w/w) cholesterol (MC) |
| 2 (8) | MC+2% (w/w) PhytoNic |
| 3 (8) | MC+ equivalent amount of phytosterols (1.5% w/w) which group 2 receives |
| 4 (8) | MC+ equivalent amount of nicotinic acid (0.5% w/w) which group 2 receives |
| 5 (8) | MC+ equivalent amount of free phytosterols (1.5% w/w) and free nicotinic acid (0.5% w/w) as group 2 receives |

**Biochemical and histological assessments:** Plasma cholesterol levels were estimated at baseline and at 4-6 week intervals during the experiment, and at the end of the experiment as previously described (13-16). Aliquots of plasma samples were used for determination of nicotinic acid or its metabolite nicotinamide levels and plant sterol composition using gas chromatography techniques. Aortic root sections were used for evaluation of atherosclerotic lesion development as previously described (13-14)

### A: Plasma total cholesterol levels

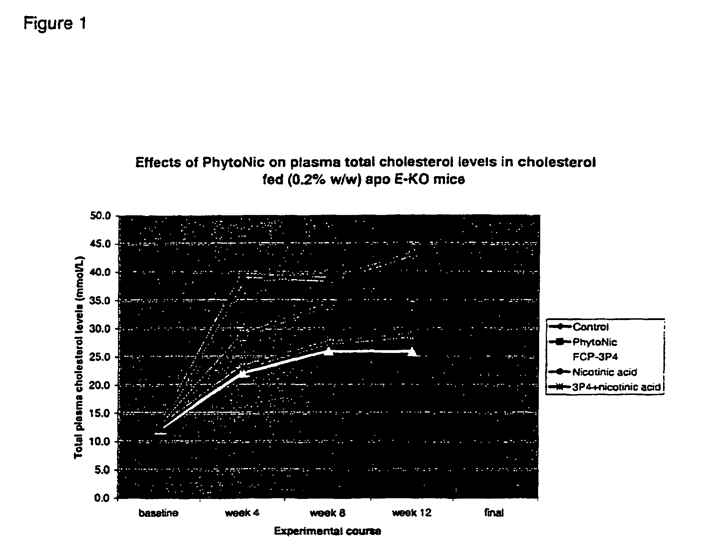

Figure 1 shows the effects of PhytoNic and its parent agents on plasma total cholesterol levels. Total cholesterol were measured in clinical lab at St. Paul's Hospital. The results indicate a cholesterol lowering effect for PhytoNic at week four. The phytosterols (a mix of beta-sitosterol, campesterol, campestanol and sitostanol referred to internally as "FCP-3P4") had a consistent cholesterol-lowering effects over the 12 weeks of study period. PhytoNic statistically reduced plasma total cholesterol levels up to 27% as compared to the control.

### B: Plasma triglycerides

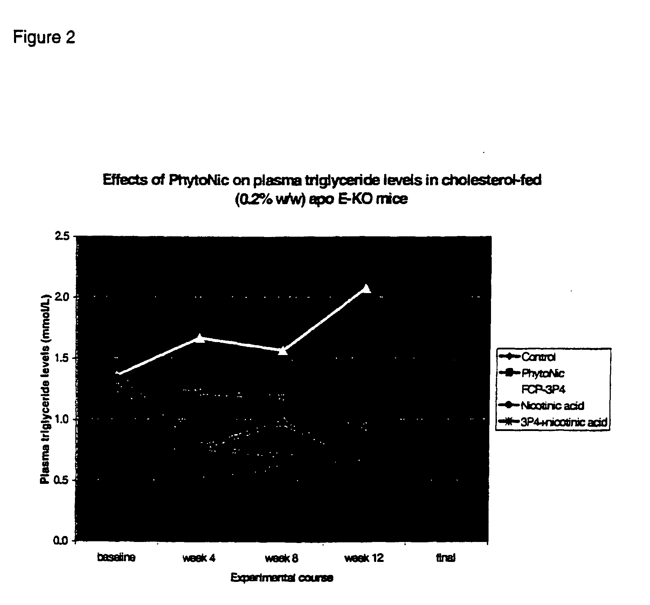

Figure 2 shows the effects of the compounds on plasma triglyceride levels. Plasma triglyceride levels were measured in clinical lab At St. Paul's Hospital. Both nicotinic acid and PhytoNic reduced triglyceride levels, but FCP-3P4 showed an increasing effect.

### C: Body Weight

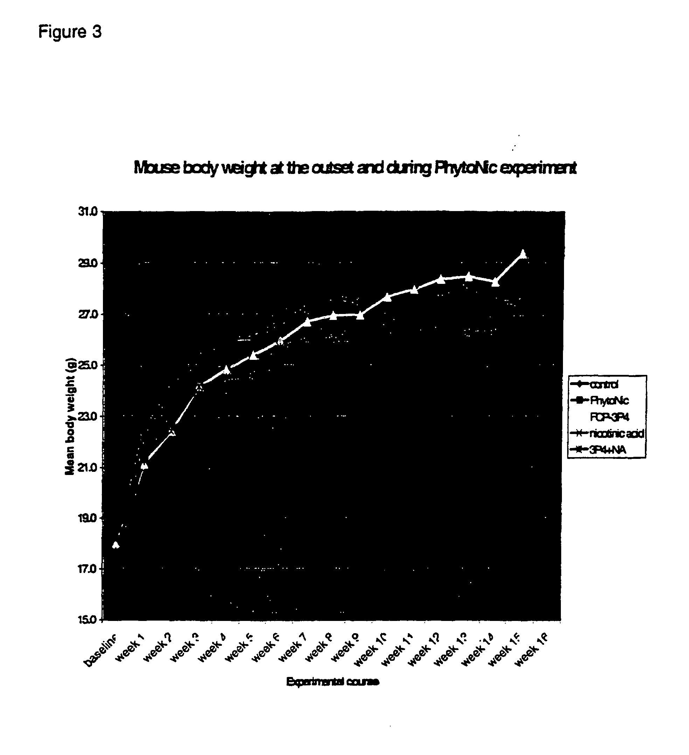

All mice similarly gained weight and had normal growth and physical appearance over the 15 weeks of the study. The results are on Figure 3.

### D: Plasma nicotinic acid and nicotinamide

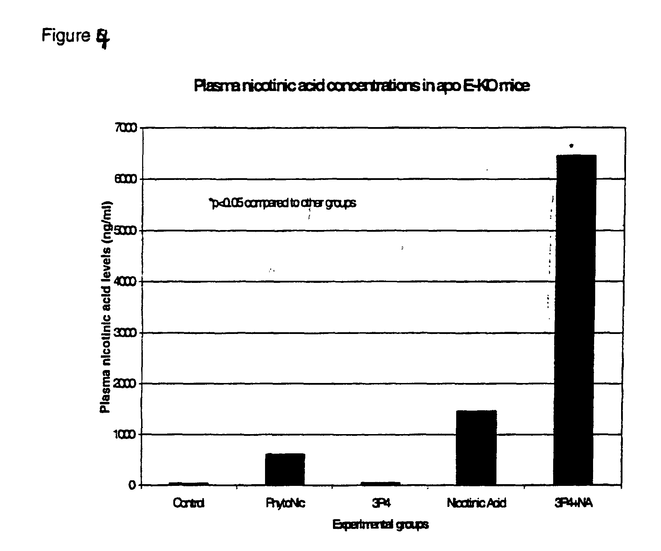

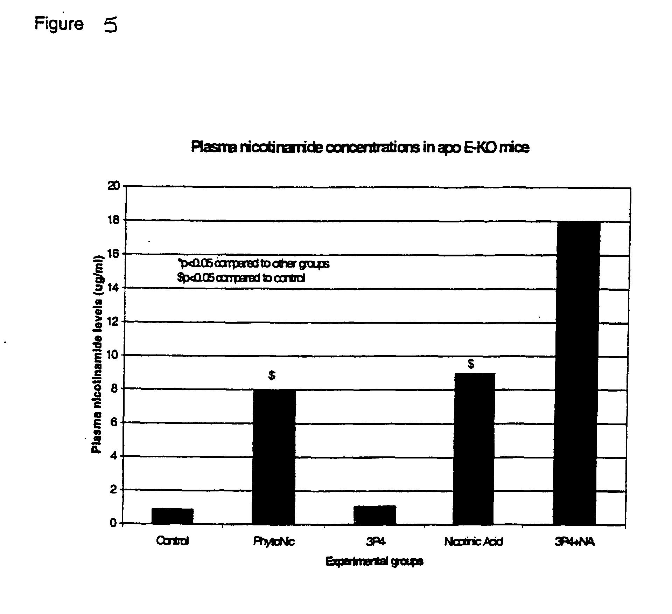

Plasma nicotinic acid and nicotinamide levels were measured at BRI under GLP conditions. PhytoNic treatment was associated with a non-statistically significant increase in plasma nicotinic acid as compared to controls. Combination of plant stanols and nicotinic acid resulted in a marked increase in the levels of both nicotinic acid and nicotinamide. The results are shown on Figures 4 and 5.

### Conclusion

PhytoNic is an effective agent in reducing plasma total cholesterol levels and was not associated with any apparent side effects. PhytoNic inhibits the triglyceride-increasing effects of its parent 3P4. Both PhytoNic and nicotinic acid have a similar decreasing effect on plasma triglyceride levels, while FCP-3P4 shows an increasing effect: Analysis of nicotinic acid and nicotinamide (shown in Figures 4 and 5) indicates that PhytoNic is not probably completely hydrolyzed in the gut. Accordingly and in consideration of all of these factors, it may be understood that PhytoNic significantly reduces toxicity of nicotinic acid while enhancing its beneficial therapeutic effects. REFERENCES
1. Law M.R., Wald N.J., Wu., Hacksaw ZA., Bailey A.; Systemic underestimation of association between serum cholesterol concentration and ischemic heart disease in observational studies: Data from BUPA Study; *Br*. *Med. J.* 1994; 308:363-366
2. Law M.R., Wald N.J., Thompson S.G.; By how much and how quickly does reduction in serum cholesterol concentration lower risk of ischemic heart disease? *Br*. *Med. J.* 1994; 308:367-373
3. La Rosa J.C., Hunninghake D.. Bush D. et al.; The cholesterol facts: A summary of the evidence relating to dietary fats, serum cholesterol and coronary heart disease:Ajoint statement by the American Heart Association and the National Heart, Lung and Blood Institute. *Circulation* 1990; 81:1721-1733
4. Havel R.J., Rapaport E.. Drug Therapy: Management of Primary Hyperlipidemia. *New England Journal of Medicine,* 1995; 332:1491-1498
5. Kuccodkar et al.; Effects of plant sterols on cholesterol metabolism. *Atherosclerosis*,
6. Lees R.S., Lees A.M. Effects of sitosterol therapy on plasma lipid and lipoprotein concentrations. In: Greten H (Ed) Lipoprotein Metabolism. Springer-Verlag, Berlin, Heidelberg, New York, 1976:119-124
7. Lees A.M., Mok H.Y.I., Lees R.S., McCluskey M.A., Grundy S.M. Plant sterols as cholesterol-lowering agents: clinical trials in patients with hypercholesterolemia and studies of sterol balance. *Atherosclerosis* 1977; 28: 325-338
8. Attwood D. Micoremulsions. In Colloidal Drug Delivery Systems (J. Kreuter ed.) Marcel Dekker, New York, 1994:32
9. Eugster C., Rivara G., Fomi G and Vai S. Marigenol Concentrates comprising Taxol andor Taxan esters as active substances. *Panmimerva Med,* 199638:234-242
10. Liposome Drug Delivery Systems, Technomic Publishing Co. Inc., Lancaster, PA 1993
11. Pharmaceutical Technology: Liposomes as Drug Delivery Systems Parts I , II and III, October 1992, November 1992 and January 1993 respectively.
12. Rajewski R.A. and Valentino J.S. Pharmaceutical Applications of Cyclodextrins/In vivo Drug Delivery System. *J. Pharm. Sci.* 1996: 85:1142-1169
13. Moghadasian et al. Pro-atherogenic and anti-atherogenic effects of probucol and phytosterols in apo E knockout mice: Possible mechanism of action. Circulation 1999:99:1733-1739.
14. Moghadasian et al. "Tall oil"-derived phytosterols reduce atherosclerosis in apo E-deficient mice. Arterioscl Thromb Vasc Biol 1997:17:119-126.
15.Moghadasian et al. Histologic, hematologic, and Biochemical characteristics of apo E-deficient mice: effects of dietary cholesterol and phytosterols. Lab Invest 1999;79:355-364.
16. Moghadasian et al. Lack of regression of atherosclerotic lesions in phytosterol-treated apo E-deficient mice. Life Sci 1999;64:1029-1036.

## Claims

1. Use of a phytosterol or phytostanol derivative having one of the following formulae: 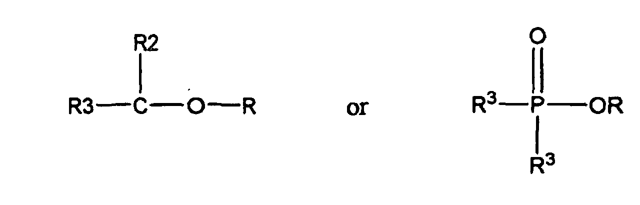 wherein R is a phytosterol or phytostanol moiety; R2 is oxygen or hydrogen (H2) and R3 comprises an aromatic group selected from benzene and substituted benzene in which substituents are C₁₋₆ alkyl and heterocyclic units comprising a ring selected from the group consisting of pyridine and substituted pyridine, optionally substituted dihydropyridine, optionally substituted piperidine, quinoline and substituted quinoline, optionally substituted dihydro- and tetrahydro- quinoline, isoquinoline and substituted isoquinoline, indole and substituted indole, imidazole and substituted imidazole, pyrrole and substituted pyrrole, furan and substituted furan and thiophene and substituted thiophene in any of which hetercyclic units optional substituents are C₁₋₆ alkyl groups, and all salts thereof, in the manufacture of a composition for use in a method of treating or preventing cardiovascular disease and its underlying conditions, including hypercholesterolemia, in an animal.

2. Use of claim 1 wherein R₃ comprises an aromatic unit which is benzene.

3. Use of claim 1 wherein R₃ comprises a heterocyclic unit.

4. Use of claim 3 wherein the heterocyclic unit comprises nicotinic acid.

5. Use of claim 1 wherein R2 is oxygen.

6. Use of claim 1 wherein R2 is hydrogen (H₂).

7. Use of claim 1 wherein the phytosterol is selected from the group consisting of sitosterol, campesterol, stigmasterol, brassicasterol, desmosterol, chalinosterol, poriferasterol and clionasterol.

8. Use of claim 1 wherein the phytostanol is selected from the group consisting of all saturated or hydrogenated phytosterols.

9. Use of claim 1 wherein the phytostanol is sitostanol.

10. Use according to claim 1 wherein the derivative is selected from the group consisting of stanol nicotinic ester and the hydrochloride salt thereof, stanol nicotinoxy acetate and di-(3-pyridinemethoxy) stanoxyphosphate.

11. Use of claim 1 wherein the animal is human.

12. A comestible or beverage which comprises a phytosterol and/or phytostanol derivative as defined in any of claims 1 to 10.

13. A phytosterol or phytostanol derivative having the formula 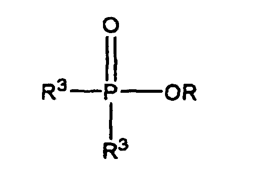 wherein R is a phytosterol or phtyostanol moiety and R3 comprises an aromatic group selected from benzene and substituted benzene in which substituents are C₁₋₆ alkyl groups or heterocyclic unit comprising a ring selected from the group consisting of pyridine or substituted pyridine, quinoline or substituted quinoline, isoquinoline or substituted isoquinoline, indole or substituted indole, imidazole or substituted imidazole, pyrrole or substituted pyrrole, furan or substituted furan and thiophene or substituted thiophene in any of which hetercyclic units optional substituents are C₁₋₆ alkyl groups; and all salts thereof.

14. A derivative according to claim 13 wherein R3 comprises a pyridyl group.

15. A compound according to claim 13 which is di(3-pyridinemethoxy)stanoxy phosphate.

## Patentansprüche

1. Verwendung eines Phytosterol- oder Phytostanol-Derivats mit einer der folgenden Formeln: 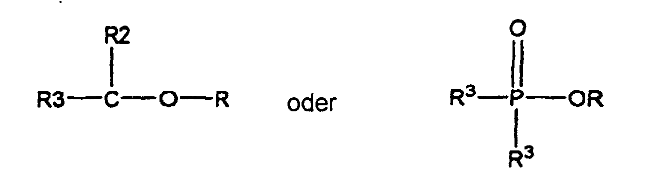 worin R eine Phyotsterol- oder Phytostanol-Einheit ist; R2 Sauerstoff oder Wasserstoff (H2) ist und R3 eine aromatische Gruppe umfasst, die ausgewählt ist aus Benzol und substituiertem Benzol, worin die Substituenten C₁₋₆-Alkyl sind, und heterocyclischen Einheiten, die einen Ring umfassen, der aus der Gruppe bestehend aus Pyridin und substituiertem Pyridin, gegebenenfalls substituiertem Dihydropyridin, gegebenenfalls substituiertem Piperidin, Chinolin und substituiertem Chinolin, gegebenenfalls substituiertem Dihydro- und Tetrahydrochinolin, Isochinolin und substituiertem Isochinolin, Indol und substituiertem Indol, Imidazol und substituiertem Imidazol, Pyrrol und substituiertem Pyrrol, Furan und substituiertem Furan und Thiophen und substituiertem Thiophen ausgewählt ist, wobei in jeder der heterocyclischen Einheiten fakultative Substituenten C₁₋₆-Alkylgruppen sind, und aller Salze derselben bei der Herstellung einer Zusammensetzung zur Verwendung in einem Verfahren zur Behandlung oder Verhütung kardiovaskulärer Krankheit und ihrer zugrundeliegenden Bedingungen, einschließlich Hypercholesterolämie, in einem Lebewesen.

2. Verwendung nach Anspruch 1, in der R3 eine aromatische Einheit umfasst, die Benzol ist.

3. Verwendung nach Anspruch 1, in der R3 eine heterocyclische Einheit umfasst.

4. Verwendung nach Anspruch 3, in der die heterocyclische Einheit Nicotinsäure umfasst.

5. Verwendung nach Anspruch 1, in der R2 Sauerstoff ist.

6. Verwendung nach Anspruch 1, in der R2 Wasserstoff (H₂) ist.

7. Verwendung nach Anspruch 1, in der das Phytosterol aus der Gruppe bestehend aus Sitosterol, Campesterol, Stigmasterol, Brassicasterol, Desmosterol, Chalinosterol, Poriferasterol und Clionasterol ausgewählt ist.

8. Verwendung nach Anspruch 1, in der das Phytostanol aus der Gruppe bestehend aus allen gesättigten oder hydrierten Phytosterolen ausgewählt ist.

9. Verwendung nach Anspruch 1, in der das Phytostanol Sitostanol ist.

10. Verwendung nach Anspruch 1, in der das Derivat aus der Gruppe bestehend aus Stanolnicotinsäureester und dessen Hydrochlorid-Salz, Stanolnicotinoxyacetat und Di-(3-pyridinmethoxy)stanoxyphosphat ausgewählt ist.

11. Verwendung nach Anspruch 1, in der das Lebewesen ein Mensch ist.

12. Nahrungsmittel oder Getränk, das ein Phytosterol- und/oder Phytostanol-Derivat, wie in irgendeinem der Ansprüche 1 bis 10 definiert, umfasst.

13. Phytosterol- oder Phytostanol-Derivat mit der Formel 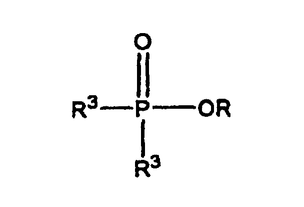 in der R eine Phytosterol- oder Phytostanol-Einheit ist und R3 eine aromatische Gruppe umfasst, die ausgewählt ist aus Benzol und substituiertem Benzol, worin die Substituenten C₁₋₆-Alkylgruppen sind, oder einer heterocyclischen Einheit, die einen Ring umfasst, der aus der Gruppe bestehend aus Pyridin oder substituiertem Pyridin, Chinolin oder substituiertem Chinolin, Isochinolin oder substituiertem Isochinolin, Indol oder substituiertem Indol, Imidazol oder substituiertem Imidazol, Pyrrol oder substituiertem Pyrrol, Furan oder substituiertem Furan und Thiophen oder substituiertem Thiophen ausgewählt ist, wobei in jeder der heterocyclischen Einheiten fakultative Substituenten C₁₋₆-Alkylgruppen sind; und alle Salze derselben.

14. Derivat nach Anspruch 13, in dem R3 eine Pyridylgruppe umfasst.

15. Verbindung nach Anspruch 13, die Di-(3-pyridinmethoxy)stanoxyphosphat ist.

## Revendications

1. Utilisation d'un dérivé de phytostérol ou de phytostanol ayant une des formules suivantes : 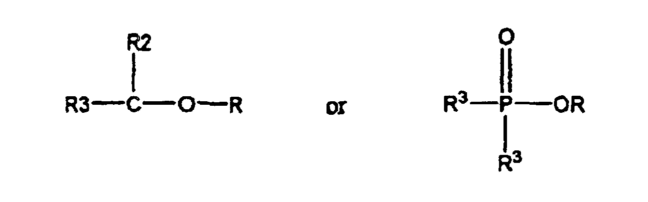 dans lequel R est une portion de phytostérol ou de phytostanol ; R2 est de l'oxygène ou de l'hydrogène (H₂) et R3 comprend un groupe aromatique choisi dans le groupe composé de benzène et de benzène substitué dans lequel les substituants sont un alkyle en C₁₋₆ et des unités hétérocycliques comprenant un anneau choisi dans le groupe consistant en pyridine et pyridine substituée, éventuellement dihydropyridine substituée, éventuellement pipéridine substituée, quinoline et quinoline substituée, éventuellement dihydro- et tétrahydro-quinoline substituée, isoquinoline et isoquinoline substituée, indole et indole substitué, imidazole et imidazole substitué, pyrrole et pyrrole substitué, furanne et furanne substitué et thiophène et thiophène substitué, dans les unités hétérocycliques desquelles les substituants éventuels sont des groupes alkyle en C₁₋₆ et tous les sels de ceux-ci, dans la fabrication d'une composition à utiliser dans un procédé de traitement ou pour éviter les maladies cardiovasculaires et leurs conditions sous-jacentes, y compris l'hypercholestérolémie, chez un animal.

2. Utilisation selon la revendication 1, dans laquelle R₃ comprend une unité aromatique qui est du benzène.

3. Utilisation selon la revendication 1, dans laquelle R₃ comprend une unité hétérocyclique.

4. Utilisation selon la revendication 3, dans lequel l'unité hétérocyclique comprend de l'acide nicotinique.

5. Utilisation selon la revendication 1, dans laquelle R2 est de l'oxygène.

6. Utilisation selon la revendication 1, dans laquelle R2 est de l'hydrogène (H₂).

7. Utilisation selon la revendication 1, dans laquelle le phytostérol est choisi dans le groupe consistant en sitostérol, campestérol, stigmastérol, brassicastérol, desmostérol, chalinostérol, poriférastérol et clionastérol.

8. Utilisation selon la revendication 1, dans laquelle le phytostanol est choisi dans le groupe consistant en tous les phytostérols saturés ou hydrogénés.

9. Utilisation selon la revendication 1, dans laquelle le phytostanol est du sitostanol.

10. Utilisation selon la revendication 1, dans laquelle le dérivé est choisi dans le groupe consistant en ester nicotinique de stanol et son sel hydrochlorure, l'acétate nicotinoxy de stanol et le di-(3-pyridineméthoxy)-stanoxyphosphate.

11. Utilisation selon la revendication 1, dans laquelle l'animal est un être humain.

12. Produit comestible ou boisson comprenant un dérivé de phytostérol et/ou de phytostanol, comme défini dans n'importe laquelle des revendications 1 à 10.

13. Dérivé de phytostérol ou de phytostanol ayant la formule 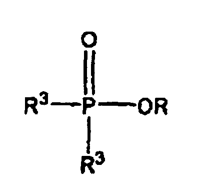 dans lequel R est une portion de phytostérol ou de phytostanol et R3 comprend un groupe aromatique choisi à partir de benzène et de benzène substitué dans lequel les substituants sont des groupes alkyle en C₁₋₆ ou une unité hétérocyclique comprenant un anneau choisi dans le groupe consistant en pyridine ou pyridine substituée, quinoline ou quinoline substituée, isoquinoline ou isoquinoline substituée, indole ou indole substitué, imidazole ou imidazole substitué, pyrrole ou pyrrole substitué, furanne ou furanne substitué et thiophène ou thiophène substitué dans les unités hétérocycliques desquels les substituants optionnels sont des groupes alkyle en C₁₋₆ ; et tous leurs sels.

14. Dérivé selon la revendication 13, dans lequel R3 comprend un groupe pyridyle.

15. Composé selon la revendication 13, qui est un phosphate de di(3-pyridineméthoxy)stanoxy.
